# EUROPÄISCHE PATENTSCHRIFT

(11) **EP 1 272 153 B1**
(45) Veröffentlichungstag und Bekanntmachung des Hinweises auf die Patenterteilung: **07.02.2007**
(21) Anmeldenummer: 01936167.4
(22) Anmeldetag: 31.03.2001
(51) Int. Cl.: A61Q 11/00

(54) **BELAGHEMMENDES FLÜSSIGES ZAHNREINIGUNGSGEL**
PLAQUE-CONTROLLING LIQUID TOOTH CLEANING GEL
GEL DENTIFRICE LIQUIDE ANTIPLAQUE

(30) Priorität: 11.04.2000 DE 10017998
(43) Veröffentlichungstag der Anmeldung: 08.01.2003
(73) Patentinhaber: Henkel Kommanditgesellschaft auf Aktien, 40589 Düsseldorf (DE)
(72) Erfinder: LEINEN, Hans, Theo, 40229 Düsseldorf (DE); GREGORI, Dorothea, 41470 Neuss (DE); WÜLKNITZ, Peter, 42799 Leichlingen (DE)
(86) Internationale Anmeldenummer: PCT/EP2001/003697
(87) Internationale Veröffentlichungsnummer: WO 2001/076547

(56) Entgegenhaltungen:
- DE-A- 2 134 862
- DE-A- 2 829 037
- FR-A- 2 150 914
- GB-A- 1 349 373

## Beschreibung

Die Erfindung betrifft flüssige Zahnreinigungsmittel mit einem Gehalt an Poliermitteln, Feuchthaltemitteln und Wasser, die als belaghemmende Kompontente ein antimikrobielles 5-Amino-hexahydropyrimidin enthalten.

Das 1.3-Bis-(2-ethylhexyl)-5-methyl-5-amino-hexahydropyridin ist als antiseptische Komponente mit der Bezeichnung Hexetidin auch in Mund- und Zahnpflegemitteln bekannt, z.B. aus den Patentanmeldungen GB 2001526 A1, EP 0 229 375 A1, DE 3729401 A1 und EP 0 408 174 A1.

Der genannte Stand der Technik offenbart aber keine flüssigen Zahnreinigungsmittel, die diese belaghemmende Komponente in einer Poliermittel enthaltenden Formulierung enthalten. Die Herstellung fließfähiger Zahnreinigungsmittel mit darin dispergierten Polierkomponenten stellt hohe Anforderungen an die Stabilität solcher Systeme gegen Sedimentation. Ein weiteres Problem besteht darin, solchen Zubereitungen belaghemmende Wirkstoffe einzuarbeiten, ohne daß die Transparenz und die Wirksamkeit der Wirkstoffe beeinträchtigt wird. Schließlich sollen solche Zahnreinigungsmittel auch weitere Kompontenen enthalten, die einer Demineralisierung des Zahnschmelzes entgegenwirken.

Gegenstand der Erfindung ist ein wäßriges, flüssiges Zahnreinigungsgel mit einer Viskosität unterhalb 50 Pa.s (20°C), gekennzeichnet durch einen Gehalt von
- 10-20 Gew.-%: eines Kieselsäue-Poliermittels
- 40-60 Gew.-%: eines Feuchthaltemittels, ausgewählt aus Sorbit, Glycerin, Polyethylenglycol und Gemischen davon
- 0,01-0,2 Gew.-%: Magnesium in Form eines gelösten Salzes und
- 0,01-1 Gew.-%: eines antimikrobiellen 5-Amino-Hexahydropyrimidins

Als flüssig im Sinne der Erfindung, werden dabei solche Zubereitungen verstanden, deren Viskosität insbesondere unter 40 m Pa·s (bei 20°C und bei einer Schergeschwindigkeit von D=4s⁻¹, bei Messung in einem Rotationsviskosimeter des Typs Brookfield RVF mit Spindel 3 oder 4 r.p.m) liegt und die sich aus einem flexiblen Spenderfläschchen durch leichten Druck dosieren lassen. Durch die erfindungsgemäße Viskosität wird ein langsames Einsinken des Gels in die Borsten der Zahnbürste bewirkt.

Bevorzugt weisen die erfindungsgemäßen Zahnreinigungsgele auch eine gewisse Transparenz auf. Die Transparenz und die erfindungsgemäße Viskosität werden durch die Menge und Auswahl der verwendeten Polierkomponenten, Verdickungsmittel und Feuchthaltemittel erzielt. Es wurde festgestellt, daß durch den Gehalt des Magnesiumsalzes die Transparenz der Zusammensetzungen verbessert wird. Außerdem leistet das Magnesiumsalz einen wertvollen Beitrag zur remineralisierenden Wirkung der Zahnreinigungsmittel.

Als Kieselsäure-Polierkomponenten eignen sich alle als Putzkörper bekannten Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew.-%, so werden die sogenannten Hydrogelkieselsäuren erhalten, wie sie z.B. aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sog. Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% des Zahngels. Bevorzugt geeignete Fällungskieselsäuren dieser Art weisen außerdem gerundete Ecken und Kanten auf und sind unter der Handelsbezeichnung Sident^{®} 12 DS (DEGUSSA) erhältlich. Eine weitere geeignete Kieselsäure ist Zeodent 113 (Huber Corp) mit einer BET-Oberfläche von 150-250 m²/g.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 - 14 µm bei einer spezifischen Oberfläche von 40 - 75 m²/g (nach BET) aus und eignen sich besonders gut für flüssige Zahngele gemäß der vorliegenden Erfindung.

Weitere Poliermittel, insbesondere solche, die für die Transparenz der Zusammensetzungen abträglich sind, aber bereits in kleinen Anteilen die Reinigugnsleistung erheblich steigern, sind gegebenenfalls in Mengen von weniger als 2 Gew.-% enthalten. Solche Poliermittelkomponenten sind z.B. Bims, Zirkoniumsilikat oder Gamma-Aluminiumoxid.

Von entscheidender Bedeutung für die Rheologie und Transparenz der erfindungsgemäßen flüssigen Zahnreinigungsgele ist die Zusammensetzung der flüssigen Trägerphase aus Wasser und Feuchthaltemitteln. Die Gesamtmenge von 40-60 Gew.-% der Feuchthaltemittel-Kombination bezieht sich auf das gesamte Zahnreinigungsgel und besteht im wesentlichen aus Sorbit, Glycerin und Polyethylenglycol. Als Polyethylenglycol können sowohl niedermolekulare als auch hochmolekulare Polyethylenglycole, also solche mit mittleren Molekulargewichten von 200 -10 000 Verwendung finden.

Generell sollte bei Verwendung hoher Anteile an Sorbit und niedriger Anteile an Glycerin ein höhermolekulares Polyethylenglycol eingesetzt werden. Bevorzugt liegen die Feuchthaltemittel Sorbit (a), Glycerin (b) und Polyethylenglycol (c) in einem Gewichtsverhältnis von (a) : (b) : (c) = 10 : (0-8) : (0,2-2) in den erfindungsgemäßen Zahnreinigungsgelen vor.

Der Wassergehalt der erfindungsgemäßen Zahnreinigungsgele beträgt 25-35 Gew.-% und ergibt sich als Summe der durch die Rohstoffe wie z.B. das 70%-ige Sorbit oder das 86%-ige Glycerin eingetragenen und der separat zugesetzten Wassermengen.

Die angegebenen Mengen an Sorbit und Glycerin beziehen sich auf eine wasserfreie Aktivsubstanz. Als wasserlösliches Magnesiumsalz eignen sich sowohl anorganische Salze wie z.B. MgCl₂, MgF₂, MgSO₄ und deren Hydrate als auch organische Magnesiumsalze wie z.B. das Magnesiumsalz der Glycerin-phosphorsäuren (Magnesium-Glycerophosphat), der Glycolsäure, der Milchsäure, der Citronensäure, der Gluconsäure, der Gutaminsäure oder der Asparaginsäure. Bevorzugt wird Magnesium in Form des MgSO₄. 7H₂O eingesetzt.

Geeignete antimikrobielle 5-Amino-hexahydropyrimidine sind z.B. aus US 2,837,463 bekannt. Ein besonderes für die Anwendung in Zahnpflegemitteln geeignetes Produkt ist das 1,3-Bis-(2-ethylhexyl)-5-methyl-5-amino-hexahydropyrimidin, das auch unter der Bezeichnung Hexetidin geläufig ist. Zur Einarbeitung in die erfindungsgemäße Zahnreinigungsgele können die 5-Amino-hexahydropyrimidine in freier Form oder auch in Form ihrer löslichen Salze, z.B. der Sulfate, Chloride, Fluoride, Hydrogenphosphate, Lactate, Glycolate, Citrate oder Salze von Organo-polyphosphonsäuren, z.B. der 1-Hydroxyethan-1,1-diphosphonsäure oder der Azacycloheptan-2,2-diphosphonsäure eingesetzt werden. Es kommt dabei lediglich darauf an, daß wenigstens 0,01 Gew.-% des 5-Amino-hexahydropyrimidins in der Zusammensetzung gelöst vorliegt.

Zusätzlich zu den genannten obligatorischen Komponenten können die erfindungsgemäßen Zahnreinigungsmittel 1-10 Gew.-% weiterer Zahnpasteninhaltsstoffe enthalten. Solche Inhaltsstoffe sind z.B. Bindemittel, Tenside, Geschmacks- und Süßstoffe, Antizahnsteinwirkstoffe, Fluorverbindungen, Vitamine, Panthenol und andere Wirksubstanzen.

Als Bindemittel können z.B. natürliche und/oder synthetische wasserlösliche Polymere wie Alginate, Carragheenate, Traganth, Stärke und Stärkeether, Celluloseether wie z.B. Carboxymethylcellulose (Na-Salz), Hydroxyethylcellulose. Methylhydroxypropylcellulose, Guar, Akaziengum, Agar-Agar, Xanthan-Gum, Succinoglycan-Gum, Johannisbrotmehl.

Pectine, wasserlösliche Carboxyvinylpolymere (z.B. Carbopol^{®}-Typen), Polyvinylalkohol, Polyvinylpyrrolidon, Polyethylenglycole, insbesondere solche mit Molekulargewichten von 1 500 - 1 000 000 eingesetzt werden.

Besonders gut geeignete Bindemittel sind Xanthan-Gum, Carboxymethylcellulose, Polyvinylpyrrolidon und Gemische dieser wasserlöslichen Polymeren, die in einer Menge bis zu 1.0 Gew.-% enthalten sein können.

Geeignete Tenside sind z.B. lineare Natriumalkylsulfate mit 12 - 18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12 - 16 C-Atomen in der linearen Alkylgruppe und 2-6 Glycolethergruppen im Molekül, von linearem Alkan(C₁₂-C₁₈)-sulfonat, von Sulfobernsteinsäuremonoalkyl(C₁₂-C₁₈)-estern, von sulfatierten Fettsäuremonoglyceriden. sulfatierten Fettsäurealkanolamiden, Sulfoessigsäurealkyl(C₁₂-C₁₆)-estern. Acylsarcosinen. Acyltauriden und Acylisethionaten mit jeweils 8 - 18 C-Atomen in der Acylgruppe.

Weiterhin eignen sich auch ampholytische und zwitterionische Tenside, insbesondere Betain-Tenside wie z.B. Kokosalkylbetain oder Kokosacylamidopropyl-Betain. Schließlich können auch nichtionische Tenside eingesetzt werden, z.B. die Oxethylate von Fettsäuremono- und Diglyceriden, von Sorbitan-Fettsäureestern und Alkyl(oligo)-glucoside.

In einer besonders bevorzugten Ausführung enthalten die erfindungsgemäßen Zahnreinigungsgele als weitere Inhaltsstoffe ein wasserlösliches Bindemittel und eine Tensidkombination aus anionischen, zwitterionischen und nichtionischen Tensiden.

Als Geschmackstoffe werden üblicherweise Aromen und Süßungsmittel verwendet. Geeignete Aromen sind z.B. Pfefferminzöl, Krauseminzöl, Eukalyptusöl, Anisol, Fenchelöl, Kümmelöl, Methylacetat, Zimtaldehyd, Anethol, Vanillin, Thymol und andere natürliche oder naturidentische ätherische Öle oder auch synthetische Geschmackstoffe.

Als Süßungsmittel eignen sich z.B. Saccharin-Natrium, Acesulfam, Aspartam, NatriumCyclamat, Stevioside, Thaumatin, Sucrose, Lactose, Maltose oder Fructose, Glycyrrhizin u.a..

Als Antizahnsteinwirkstoffe können z.B. organische Phosphonate wie z.B. Azacycloheptan-2,2-diphosphonsäure-Dinatriumsalz oder 1-Hydroxyethan-1,1-diphosphonsäure Tetranatriumsalz enthalten sein.

Als Fluorverbindungen eignen sich z.B. Natriumfluorid oder Natriummonofluorphosphat (Na₂PO₃F).

Als Vitamine können z.B. Retinol oder dessen Fettsäureester, Panthenol oder ein Salz der Pantothensäure, Ascorbinsäure oder deren Salze oder Ester oder Gemische davon enthalten sein. In einer bevorzugten Ausführung ist zur Verbesserung der entzündungshemmenden Eigenschaften eine Kombination von Panthenol und Retinol enthalten.

Weiterhin können übliche Zahnpasteninhaltsstoffe, z.B.
- Puffersubstanzen wie z.B. primäre, sekundäre oder tertiäres Alkaliphosphat, Citronensäure/Natriumcitrat
- wundheilende und entzündungshemmende Stoffe wie z.B. Harnstoff-, Allantoin, Kamillenwirkstoffe (Azulen), Alkalirhodanid, Acetylsalicylsäure-Derivate,
- niedere Alkohole wie z.B. Ethanol oder Isopropanol,
- Farbstoffe, Pigmente
- Konservierungsmittel wie z.B. Benzoesäure, Sorbinsäure und deren Salze, p-Hydroxybenzoesäure-Ester.

Die folgenden Beispiele sollen den Gegenstand der Erfindung näher erläutern:

### Beispiele

Es wurden flüssige Zahnreinigungsgele der folgenden Zusammensetzung hergestellt:

| | 1 | 2 | 3 | 4 | 5 |
|---|---|---|---|---|---|
| Sident 12 SPLS | 12 | --- | --- | 12 | --- |
| Sident 8 | --- | 12 | 12 | --- | --- |
| Sorbosil AC 39 | --- | --- | --- | --- | 12 |
| Sorbit (70%-ig) | 30 | 30 | 70 | 75 | 30 |
| Glycerin (86%-ig) | 33 | 33 | --- | --- | 33 |
| Lipoxol 1550 | 1 | 1 | 1,5 | --- | 1,0 |
| Lipoxol 400 | --- | --- | --- | 2,0 | --- |
| Keltrol F | 0,33 | 0,5 | --- | --- | 0,5 |
| Cekol 2000 | --- | --- | 0,5 | 0,2 | --- |
| MgSO₄·7H₂O | 0,21 | 0,21 | 0,5 | 0,3 | 0,21 |
| ZnSO₄·7H₂O | 0,16 | 0,16 | 0,16 | --- | 0,16 |
| Mn SO4·H₂O | 0,01 | 0,01 | --- | --- | 0,01 |
| Texapon K1296 | 1,5 | 1,5 | 1,5 | 1,5 | 1,5 |
| Tego Betain BL 215 | 0,6 | 0,6 | 0,6 | 0,6 | 0,6 |
| Tagat S | 0,5 | 0,5 | 0,5 | 0,5 | 0,5 |
| Hexetidin | 0,03 | 0,03 | 0,05 | 0,04 | 0,03 |
| Triclosan | --- | --- | --- | --- | 0,10 |
| Na F | 0,33 | 0,33 | --- | --- | 0,32 |
| Na₂PO₃F | --- | --- | 1,1 | 1,0 | --- |
| Na₂HPO₄ | 0,2 | 0.2 | 0.2 | 0,2 | 0.2 |
| Na-Benzoat | 0.5 | 0,5 | --- | --- | 0,5 |
| Na-Saccharinat | 0.2 | 0,2 | 0,2 | 0,2 | 0,2 |
| Aromaöl | 1,0 | 1,0 | 1,0 | 1,0 | 1,0 |
| Ethanol, Farbstoff | 2,0 | 2,0 | 2.0 | 2,0 | 2,0 |
| Wasser | ad 100 | ad100 | ad100 | ad100 | ad 100 |

Es wurden die folgenden Handelsprodukte verwendet:

| | |
|---|---|
| Sident 12 SPLS: | Fällungskieselsäure |
| (Degussa-Hüls) | Spez. Oberfläche (BET): 80 m²/g |
| | |
| Sident 8: | Fällungskieselsäure |
| (Degussa-Hüls) | Spez. Oberfläche (BET): 60 m²/g |
| | |
| Sorbosil AC39: | Fällungskieselsäure |
| (Crosfield) | |
| | |
| Lipoxol 1550: | Polyethylenglycol, MG 1550 |
| (RWE/DEA) | |
| | |
| Lipoxol 400: | Polyethylenglycol, MG 400 |
| (RWE/DEA) | |
| | |
| Keltrol F: | Xanthan Gum |
| (Kelco) | |
| | |
| Cekol 2000: | Natrium-Carboxymethylcellulose |
| (Metsa) | |
| | |
| Texapon K1296: | Natrium-Laurylsulfat (Pulver) |
| (Cognis Deutschland) | |
| | |
| Tego-Betain BL 215: | Fettsäureamidoalkylbetain (30%-ig) |
| (Goldschmidt) | (Cocoamidopropyl Betain) |
| | |
| Tagat S: | PEG-30 Glyceryl Stearat |
| (Goldschmidt) | |

## Patentansprüche

1. Wäßriges flüssiges Zahnreinigungsgel mit einer Viskosität unterhalb 50 Pa.s (20°C), **gekennzeichnet durch** einen Gehalt von
10-20 Gew.-% eines Kieselsäure-Poliermittels
40-60 Gew.-% eines Feuchthaltemittels ausgewählt aus Sorbit, Glycerin. Polyethylenglycol und Gemischen davon
0,01-0,2 Gew.-% Magnesium in Form eines gelösten Salzes und
0.01-1 Gew.-% eines antimikrobiellen 5-Amino-hexahydropyrimidins

2. Flüssiges Zahnreinigungsgel gemäß Anspruch 1, **dadurch gekennzeichnet, daß** zusätzlich ein wasserlösliches Bindemittel und eine Tensidkombination aus anionischen, zwitterionischen und nichtionischen Tensiden enthalten ist.

3. Flüssiges Zahnreinigungsgel gemäß einem der Ansprüche 1 oder 2, **dadurch gekennzeichnet, daß** als Bindemittel Xanthan-Gum, Carboxymethylcellulose, Polyvinylpyrrolidon oder Gemische dieser wasserlöslichen Polymeren in einer Menge bis zu 1 Gew.-% enthalten sind.

## Claims

1. A water-based liquid tooth cleaning gel with a viscosity below 50 Pa·s (20°C), **characterized by** a content of
10 to 20% by weight of a silica polish,
40 to 60% by weight of a humectant selected from sorbitol, glycerol, polyethylene glycol and mixtures thereof,
0.01 to 0.2% by weight of magnesium in the form of a dissolved salt and
0.01 to 1% by weight of an antimicrobial 5-aminohexahydropyrimidine.

2. A liquid tooth cleaning gel as claimed in claim 1, **characterized in that** a water-soluble binder and a surfactant combination of anionic, zwitterionic and nonionic surfactants are additionally present.

3. A liquid tooth cleaning gel as claimed in claim 1 or claim 2, **characterized in that** xanthan gum, carboxymethyl cellulose, polyvinyl pyrrolidone or mixtures of these water-soluble polymers is/are present as binder in a quantity of up to 1% by weight.

## Revendications

1. Gel aqueux liquide destiné au nettoyage des dents, présentant une viscosité inférieure à 50 Pa.s (20°C), **caractérisé par** une teneur de
10-20% en poids d'un agent de polissage à base d'acide silicique
40-60% en poids d'un agent de rétention de l'humidité choisi parmi le sorbitol, le glycérol, le polyéthylèneglycol et les mélanges de ceux-ci
0,01-0,2% en poids de magnésium sous forme d'un sel dissous et
0,01-1 % en poids d'une 5-amino-hexahydropyrimidine antimicrobienne.

2. Gel liquide destiné au nettoyage des dents selon la revendication 1, **caractérisé en ce qu'**il contient en outre un liant soluble dans l'eau et une combinaison d'agents tensioactifs constituée par des agents tensioactifs anioniques, zwittérioniques et non ioniques.

3. Gel liquide destiné au nettoyage des dents selon l'une quelconque des revendications 1 ou 2, **caractérisé en ce qu'**il contient comme liant de la gomme de xanthane, de la carboxyméthylcellulose, de la polyvinylpyrrolidone ou des mélanges de ces polymères solubles dans l'eau en une quantité allant jusqu'à 1% en poids.
